(19) Europäisches Patentamt
European Patent Office
Office européen des brevets

(11) Veröffentlichungsnummer: **0 287 659**
**A1**

(12) # EUROPÄISCHE PATENTANMELDUNG

Veröffentlicht nach Art. 158 Abs. 3 EPÜ

(21) Anmeldenummer: **87900349.9**

(51) Int. Cl.³: **C 07 F 7/10**

(22) Anmeldetag: **21.10.86**

Daten der zugrundeliegenden internationalen Anmeldung:

(86) Internationale Anmeldenummer:
**PCT/SU86/00104**

(87) Internationale Veröffentlichungsnummer:
**WO88/03143 (05.05.88 88/10)**

(43) Veröffentlichungstag der Anmeldung:
**26.10.88 Patentblatt 88/43**

(84) Benannte Vertragsstaaten:
**CH DE FR GB IT LI**

(71) Anmelder: **INSTITUT ORGANICHESKOGO SINTEZA AKADEMII NAUK LATVIISKOI SSR**
**ul. Aizkraukles, 21**
**Riga 226006(SU)**

(72) Erfinder: **LUKEVITS, Edmund Yanovich**
**ul. Ierikju, 43-10**
**Riga, 226059(SU)**

(72) Erfinder: **KASTRON, Valeria Vasilievna**
**ul. Avotu, 33-2**
**Riga, 226009(SU)**

(72) Erfinder: **VITOLIN, Rasma Oskarovna**
**ul. Suvorova, 117-13**
**Riga, 226001(SU)**

(72) Erfinder: **ERCHAK, Nikolai Petrovich**
**ul. Dzirnavu, 161-13**
**Riga, 226018(SU)**

(72) Erfinder: **SKRASTINSH, Indulis Petrovich**
**ul. Lienes, 9-26**
**Riga, 226009(SU)**

(72) Erfinder: **DUBUR, Gunar Yanovich**
**ul. Ierikju, 43-2**
**Riga, 226059(SU)**

(72) Erfinder: **KIMENIS, Agris Adolfovich**
**ul. Staitseles, 15-208**
**Riga, 226084(SU)**

(74) Vertreter: **von Füner, Alexander, Dr. et al,**
**Patentanwälte v. Füner, Ebbinghaus, Finck Mariahilfplatz 2 & 3 Postfach 95 01 60**
**D-8000 München 95(DE)**

(54) **2,6-DIMETHYL-3,5-DIMETHOXYKARBONYL-4-(5'-TRIMETHYLSILYL-2'-FURYL)-1,4-DIHYDROPYRIDINE.**

(57) 2,6-Dimethyl-3,5-dimethoxykarbonyl-4-(5'-trimethylsilyl-2'-furyl)-1,4-dihydropyridin wird durch folgende Formel gekennzeichnet :

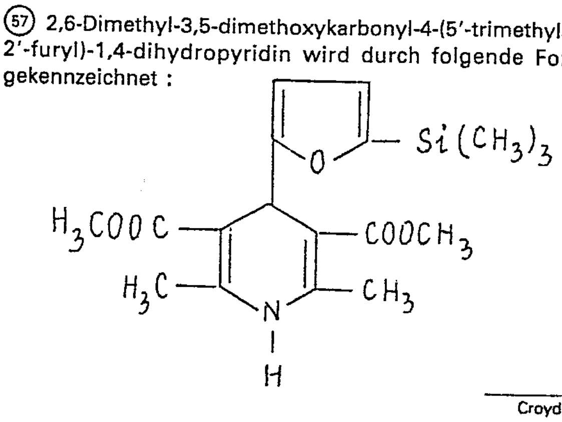

Diese Verbindung weist eine hypotensive Wirkung auf.



Croydon Printing Company Ltd.

EPAA-36540.2

# 2,6-DIMETHYL-3,5-DIMETHOXYKARBONYL-4-(5'-TRIMETHYLSILYL-2'-FURYL)-I,4-DIHYDROPYRIDIN

## Gebiet der Technik

Die vorliegende Erfindung bezieht sich auf das Gebiet der organischen Synthese, sie betrifft die Derivate des I,4-Dihydropyridins und insbesondere das 2,6-Dimethyl-3,5-dimethoxykarbonyl-4-(5)-trimethylsilyl-2'-furyl)-I,4-dihydropyridin.

Die I,4-Dihydropyridin-Derivate weisen hypotensive und koronarerweiternde Wirkung auf, und einige davon werden in der Medizin verwendet.

## Vorhergehender Stand der Technik

Ein besonders bekanntes Derivat des I,4-Dihydropyridins ist das 2,6-Dimethyl-3,5-dimethoxykarbonyl-4-(o-nitrophenyl)-I,4-dihydropyridin (Nifedipin), das hypotensive und koronarerweiternde Wirkung (Arzneim.-Forsch., v. 32, 1982, E.Kusano, J.Asano, K.Takeda, J.Matsumoto, A.Ebihara. "Hypotensive effect of Nifedipine in Hypertensive Patients with Chronic Renal Failure", pp. 1575-1580) aufweist.

Obwohl Nifedipin in der medizinischen Praxis umfassend verwendet wird, ist es jedoch nicht lichtbeständig und ist hoch toxisch.

Bekannte Derivate des I,4-Dihydropyridins, die Silizium in der 3,5-Estergruppe aufweisen, stehen in ihrer hypotensiven Wirkung beträchtlich dem Nifedipin nach (Eur.J.Med.Chem., v. 18, No 2, 1983, R.Tacke, A.Bentlage, R.Towart, E.Möller. "Sila-analogues of Nifedipine-like dialkyl 2,6-dimethyl-4-aryl-I,4-dihydropyridine-3,5-dicarboxylates", pp. 155-161).

Von den Derivaten des I,4-Dihydropyridins, bekannt ist das 2,6-Dimethyl-3,5-dimethoxykarbonyl-4-(2)-furyl)-I,4-dihydropyridin, das ebenfalls eine hypotensive Wirkung aufweist, die aber im Vergleich zum Nifedipin bedeutend niedriger ist. /Khimiko-farmatsevtichesky zhurnal, Nr. 6, 1979, V.V. Kastron, G.J. Dubur, R.O.Vitolin, A.A.Kimenis; "Sintez i farmakologicheskaya aktivnost 4-Furyl-I,4-dihydropyridinov, pp. 57-62/.

Die obengenannten Derivate des I,4-Dihydropyridins weisen zwar eine hypotensive Wirkung auf, diese Wirkung ist jedoch nicht prolongiert, was seine mehrmalige Verabreichung im Verlaufe von 24 Stunden erforderlich macht. Das Nifedipin besitzt ausserdem eine hohe Toxizität.

## Offenbarung der Erfindung

Die Aufgabe der vorliegenden Erfindung bestand in der Suche nach einem solchen Derivat des I,4-Dihydropyridins, das eine prolongierte hypotensive Wirkung aufweist und dabei eine niedrige Toxizität besitzt.

Die gestellte Aufgabe wurde durch die Entwicklung eines Derivates des I,4-Dihydropyridins gelöst, als solches tritt, erfindungsgemäss, 2,6-Dimethyl-3,5-dimethoxykarbonyl-4-(5'-trimethylsilyl-2'-furyl)-I,4-dihydropyridin folgender Formel auf

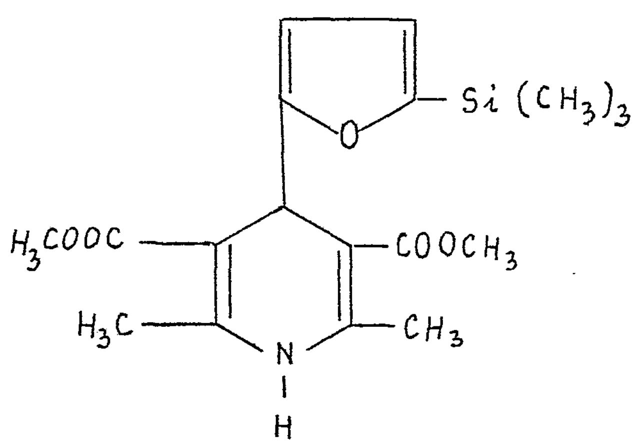

Diese Verbringung ist neu. Von uns wurde festgestellt, dass die genannte Verbindung eine prolongierte hypotensive Wirkung aufweist und eine relativ niedrige Toxizität hat.

Die genannte Verbindung stellt eine kristalline Substanz von geblichem Farbton vom Schmelzpunkt 158-159°C dar. Sie lässt sich gut im Methyl- und Ethylalkohol, im Chloroform und Azeton lösen und ist im Wasser praktisch unlöslich. Diese Verbindung ist sowohl im kristallinen Zustand als auch in Form von Lösungen stabil. In fester Form bei Lagerung in Licht verändert diese Verbindung ihren UV-Spektrum innerhalb eines Jahres nicht. In einer Alkohollösung mit einer Konzentration von $5.10^{-5}$ Mol veränderte sich der UV-Spektrum ebenfalls nicht innerhalb von 6 Monaten. Dagegen im UV-Spektrum des Nifedipins wird bereits in 6 Stunden unter ähnlichen Bedingungen des Auftreten des Maximums an der oxydischen Form des Nifedipins nachgewiesen.

Die genannte Verbindung wird in einem Verfahren hergestellt, das die Kondensation des Azetessigsäuremethylesters, des Ammoniaks und des 5-Trimethylsilylfurfurols in einer Methylalkohollösung vorsieht. Diese Verbindung kann ausserdem durch Kondensation des Azetessigsäuremethylesters, des β-Aminokrotonsäuremethylesters und des 5-Trimethylsilylfurfurols im Medium eines organischen Lösungsmittels hergestelt werden. Die Reaktion führt man bei einem normalen Druck unter Erwärmung durch. Man erhält ein Produkt mit einer guten Ausbeute.

Die Ausgangskomponenten, die bei der Herstellung der genannten Verbindung zum Einsatz kommen, stellen bekannte und zugähngliche Stoffe dar.

Farmakologische Untersuchung der erfindungsgemässen Verbindung wurde im Vergleich zum in der klinischen Praxis verwendeten Präparat der gleichen Reihe, zum Nifedipin, durchgeführt.

In Versuchen an spontan-hypertensiven Ratten verursachte diese Verbindung bei einer einmaligen Verabreichung (10 mg/kg in den Magen) die Senkung des systolischen arteriellen Blutdruckes. Der maximale hypotensive Effekt (zu 21%) wurde in der sechsten Stunde nach der Verabreichung der Substanz beobachtet, in 24 Stunden wurde der Blutdruck um weitere 17% im Vergleich zum Ausgangsblutdruck

herabgesetzt. Die einmalige Verabreichung der Verbindung innerhalb von 5 Tagen in einer Dosis von 10 mg/kg in den Magen verursachte die maximale Senkung des Blutdruckes (um 22% ) am 6.Tag, das heisst I Tag nach der Einstellung der Verabreichung der Substanz. Der erniedrigte Blutdruck wurde am dritten Tag (um 10%) nach der Einstellung der Verabreichung der Substanz nachgewiesen. Im Vergleich zum Nifedipin ist zu ersehen, dass das letztere die Verbindung nach I Stunde nach der Verabreichung übertrifft, gegen die 6.Stunde erweisen beide Verbindungen die gleiche hypotensive Wirkung und in 24 Stunden erweist die erfindungsgemässe Verbindung immernoch eine hypotensive Wirkung, während bei Ratten, denen das Nifedipin verabreicht wurde, der systolische Blutdruck auf den Ausgangsblutdruck zurückgeht. Somit übertrifft die erfindungsgemässe Verbindung das Nifedipin in der prolongierten Wirkung sowohl bei einmaliger als auch bei wiederholter Verabreichung und sie erweist eine weniger ausgeprägte Wirkung auf die Herzschlagfrequenz. Die Angaben der Untersuchungen sind in der Tabelle I angeführt. In dieser Tabelle ist zum Vergleich die Verbindung 2,6-Dimethyl-3,5-dimethoxykarbonyl-4-(2'-furyl)-I,4-dihydropyridin angeführt, das ein Analogon für die erfindungsgemässe Verbindung nach ihrer chemischen Struktur darstellt.

Tabelle I

Einfluss der Verbindungen auf den arteriellen Blutdruck in Versuchen an spontan-hypertensiven Ratten

| Verbindung | Dosis, mg/kg | Hypotensive Wirkung, % | | | |
|---|---|---|---|---|---|
| | | in I Stunde | in 6 Stunden | in 24 Stunden | in 48 Stunden |
| Erfindungsgemässe Verbindung | 10 | 13 | 21 | 17 | - |
| | 20 | - | 30 | 39 | 28 |
| Analogon | 10 | 12 | 7 | 0 | 0 |
| Nifedipin | 10 | 34 | 23 | 0 | 0 |

Wie aus der Tabelle I zu ersehen ist, weist die erfindungsgemässe Verbindung eine prolongiertere Wirkung als das Analogon und das Nifedipin auf.

Von uns wurde festgestellt, dass die genannte Verbindung in einer Dosis von IO mg/kg und 20 mg/kg in Versuchen an spontan-hypertensiven Ratten (SHR) bei der peroralen Verabreichung die Herzschlagfrequenz herabsetzt beziehungsweise nicht verändert. In dieser Wirkung unterscheidet sich unsere Verbindung vorteilhaft vom Nifedipin, das Tachykardie hervorruft.

Die akute Toxizität der Verbindung, des Nifedipins und des Analogons wurde an weissen rassenlosen Mäusen bei intraperitonealer Verabreichung untersucht. Die Mäuse beobachtete man während IO Tage. Die durchschnittliche Dosis letalis ($LD_{50}$) ermittelte man nach Litchfild- und Wilkokson-Methode. Die Verabreichung der genannten Verbindung in einer Dosis von höchstens IOOO mg/kg verursachte keinen Tod der Mäuse. Die Angaben der Untersuchung der akuten Toxizität sind in der Tabelle 2 angeführt.

Tabelle 2

Akute Toxizität

| Verbindung | $LD_{50}$, mg/kg |
|---|---|
| Erfindungsgemässe Verbindung | >IOOO |
| Analogon | 2900 (2304-3654) |
| Nifedipin | I85 (II9-287) |

Aus der Tabelle 2 ist zu ersehen, dass die erfindungsgemässe Verbindung eine niedrige Toxizität im Vergleich zum Nifedipin aufweist. Obwohl das Analogon auch eine niedrige Toxizität aufweist, es steht jedoch in seinen hypotensiven Eigenschaften beträchtlich nach.

Unter Berücksichtigung der ausgeprägten und prolongierten hypotensiven Wirkung der Verbindung sowie ihrer niedrigen Toxizität und hohen Stabilität im Vergleich

zum bekannten Präparat, Nifedipin, kann diese Verbindung für die Behandlung der Hypertonie verwendet werden. Die Dauer der Wirkung der Substanz lässt es voraussetzen, dass der therapeutische Effekt bei einer einmaligen Verabreichung der Substanz innerhalb von 24 Stunden erreicht werden kann.

Beste Ausführungsvariante der Erfindung

Die obengenannte Verbindung, 2,6-Dimethyl-3,5-dimethoxykarbonyl-4-(5'-trimethylsilyl-2'-furyl)-I,4-dihydropyridin, wird es empfohlen, in einem Verfahren herzustellen, das die Kondensation des Azetessigsäuremethylesters, des $\beta$-Aminikrotonsäuremethylesters und des 5-Trimethylsilylfurfurols im Medium des Methylalkohols vorsieht. Die Reaktion führt man bei der Siedetemperatur des Reaktionsgemisches und bei normalem Druck durch. Die Beendingung der Reaktion ermittelt man nach der Methode der Dünnschichtchromatografie. Nach der Beendingung der Reaktion wird das Reaktionsgemisch abgekühlt und der ausgefallene Niederschlag des Endproduktes wird isoliert. Die Ausbeute an Produkt nach der Kristallisation beträgt 61,7%. Das hergestellte Produkt entspricht der obenangeführten Formel.

Zur besseren Erläuterung der vorliegenden Erfindung wird ein Beispiel für die Herstellung der Verbindung angeführt.

Beispiel

2,45 g (I,45 mMol 5-Trimethylsilylfurfurol, I,68 g (I,45 mMol) Azetessigsäuremethylester und I,66 g (I,45 mMol) $\beta$-Aminokrotonsäuremethylester kocht man in 10 ml Methanol während 3 Stunden. Bei der Kühlung fällt ein Niederschlag von gelbem Farbton aus. Der Niederschlag wäscht man mit Hexan und man erhält 4,5 g (92,5% ) der Verbindung. Nach der Kristallisation aus Methanol erhält man 3,0 g (61,7%) des Produktes. Schmelzpunkt beträgt 158-159°C. Rf 0,70 (Hexan-Chloroform-Ethylazetat, I:I:I). UV-Spektrum, $\lambda_{max}$, Nm (lg $\varepsilon$ ):234 (4,46), 352 (3,92). IR-Spektrum, $cm^{-I}$ : I659, I7I5, 3365. PMR-Spektrum, $\delta$ ppm,

$CDCl_3$: 0,2 Singulett ($SiMe_3$); 2,I3 Singulett (2,6-Me); 3,74 Singulett (OMe); 5,25 Singulett (4'-H); 5,64 g (3'-H, Furan). J = 2 Hz; 6,45 g (4'-H, Furan). J = 2 Hz.

Gefunden %: C 59,I7; H 6,4I; N 3,84; $C_{I8}H_{25}NO_5Si$

Berechnet %: C 59,48; H 6,93; N 3,85.

## Industrielle Anwendbarkeit

Die erfindungsgemässe Verbindung, 2,6-Dimethyl-3,5-dimethoxykarbonyl-4-(5'-trimethylsilyl-2'-furyl)-I,4-dihydropyridin, kann in der pharmazeutischen Industrie für die Herstellung eines Arzneipräparates eingesetzt werden, das für die Behandlung der Hypertonie geeignet ist.

PATENTANSPRUCH

2,6-Dimethyl-3,5-dimethoxykarbonyl-4-(5'-trimethylsilyl-2'-furyl)-I,4-dihydropyridin folgender Formel:

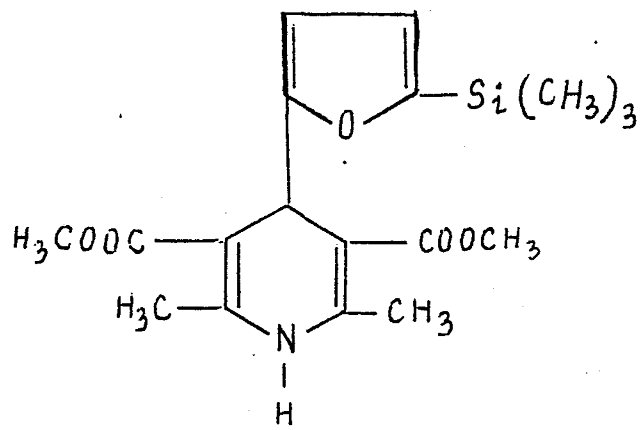

# INTERNATIONAL SEARCH REPORT

International Application No PCT/SU 86/00104

**I. CLASSIFICATION OF SUBJECT MATTER** (if several classification symbols apply, indicate all) [6]

According to International Patent Classification (IPC) or to both National Classification and IPC

$IPC^4$ - C 07 F 7/10

**II. FIELDS SEARCHED**

Minimum Documentation Searched [7]

| Classification System | Classification Symbols |
|---|---|
| $IPC^4$ | C 07 F 7/10, 7/08 |

Documentation Searched other than Minimum Documentation to the Extent that such Documents are Included in the Fields Searched [8]

**III. DOCUMENTS CONSIDERED TO BE RELEVANT** [9]

| Category * | Citation of Document, [11] with indication, where appropriate, of the relevant passages [12] | Relevant to Claim No. [13] |
|---|---|---|
| A | DE, A1,2915355, (A. Nattermann & Cie CmbH), 23 October 1980 (23.10.80), see pages 1,2 | 1 |
| A | DE, A1,2837477, (Bayer AG), 13 March 1980 (13.03.80), see pages 1-3 | 1 |
| A | CH, A5, 626375, (Rhône-Poulenc Industries), 13 November 1981 (13.11.81), see pages 1-2 | 1 |
| A | CH, A5, 542884, (Dynamit Nobel Aktiengesell-schaft), 30 November 1973 (30.11.73), see pages 1,5,6 | 1 |

* Special categories of cited documents: [10]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art.

"&" document member of the same patent family

**IV. CERTIFICATION**

| Date of the Actual Completion of the International Search | Date of Mailing of this International Search Report |
|---|---|
| 27 May 1987 (27.05.87) | 03 July 1987 (03.07.87) |
| International Searching Authority | Signature of Authorized Officer |
| ISA/SU | |